# EUROPEAN PATENT APPLICATION

(11) **EP 2 980 204 A1**
(43) Date of publication of application: **03.02.2016**
(21) Application number: 14774943.6
(22) Date of filing: 10.03.2014
(51) Int. Cl.: C12M 1/34, C12Q 1/04

(54) **MICROORGANISM DETECTION SENSOR, METHOD FOR MANUFACTURING SAME, AND POLYMER LAYER**

(30) Priority: 28.03.2013 JP 2013069739
(71) Applicant: Sharp Kabushiki Kaisha, Osaka-shi, Osaka 545-8522 (JP); Osaka Prefecture University Public Corporation, Osaka 599-8531 (JP)
(72) Inventor: IKEMIZU, Mugihei, Osaka-shi, Osaka 545-8522 (JP); TOKONAMI, Shiho, Sakai-shi Osaka 599-8531 (JP); SHIIGI, Hiroshi, Sakai-shi Osaka 599-8531 (JP); NAGAOKA, Tsutomu, Sakai-shi Osaka 599-8531 (JP)
(74) Representative: Treeby, Philip David William
(86) International application number: PCT/JP2014/056143
(87) International publication number: WO 2014/156584

(57) **Abstract**

The present invention is a sensor for detecting a microorganism, which is provided with a detection unit (17) equipped with a detection electrode (11) and a polymer layer (14), wherein the polymer layer (14) is arranged on the detection electrode and is provided with a template having a three-dimensional structure complementary to a three-dimensional structure of a microorganism (13) to be detected. The sensor detects a microorganism on the basis of the captured state of the microorganism onto the template (15). The polymer layer (14) is formed by a manufacturing method including a polymerization step (St1) of polymerizing a monomer in the presence of the microorganism (13) to be detected to form a polymer layer (14) having the microorganism (13) incorporated therein on the detection electrode, and a disruption step (St2) of bringing at least a part of the microorganism (13) incorporated in the polymer layer (14) into contact with a solution containing a lytic enzyme to disrupt the microorganism.

## Description

### TECHNICAL FIELD

The present invention relates to a sensor for detecting a microorganism, a method for manufacturing the same, and a polymer layer.

### BACKGROUND ART

In recent years, microorganism detection attracts interest in a health care industry, a food industry, agriculture, a livestock industry, aquaculture, a water factory, and the like. A contaminating microorganism which is present in food, drug, pesticide, and the like may significantly affect a human health even with a very small quantity. Moreover, microbial contamination in a hospital and an aged care facility has been a social problem. Further, as can be seen in a distribution of and a rise in demand for various antimicrobial products, a hygiene management in a general household also attracts interest. For example, in the case of a food processing factory, a bacteriological examination with samples of food to be shipped or a bacteriological examination in an environment of a factory are performed. However, in the case of measurement by means of cultivation, it requires twenty-four hours to forty-eight hours to obtain a result, and it may become a factor for a rise in a storage cost before the shipment. Therefore, a quick detection procedure is required. Moreover, also in the field of agriculture, a risk of occurrence of a disease rises as the bacterial count in a culture solution of hydroponics increases. Therefore, a quick detection procedure is effective since a measure such as disinfection can be taken promptly by grasping the bacterial count in an early stage.

In view of such a situation, a need for a technique to detect microbial contamination in a simple manner has been rising rapidly. Moreover, in a medical site, since it would be necessary to quickly specify a pathogen of a cause of an infectious disease, a technique capable of detecting a pathogen promptly with a high sensitivity is required. As methods for detecting and specifying a microorganism, there exist methods such as an ELISA method, a western blotting method, and the like. For example, these are methods of detection including, for example, causing an antigen-antibody reaction between an antibody (primary antibody) and a protein particular to a microorganism, and thereafter further causing a marked second antibody to react with an antibody (primary antibody), and monitoring a chemoluminescence of the second antibody and a hydrolysis reaction of ATP.

Moreover, Japanese Patent Laying-Open No. 2009-58232 (PTD 1) discloses a method of utilizing an electrochemical property of a polymer including a molecular template to detect an anion molecule (ATP, amino acid, and the like) derived from a microorganism.

### CITATION LIST

### PATENT DOCUMENT

PTD 1: Japanese Patent Laying-Open No. 2009-58232

### SUMMARY OF INVENTION

### TECHNICAL PROBLEM

However, the methods described above are not the methods for detecting a microorganism itself. Moreover, in the ELISA method, it is necessary to produce an antibody with respect to a protein or the like particular to a microorganism, which is not easy.

An object of the present invention is to provide a new microorganism detection sensor capable of detecting a microorganism in a prompt and simple manner with a high sensitivity, a method for manufacturing the same, and a polymer layer.

### SOLUTION TO PROBLEM

The present invention is a sensor for detecting a microorganism, which is provided with a detection unit equipped with a detection electrode and a polymer layer, wherein the polymer layer is arranged on the detection electrode and is provided with a template having a three-dimensional structure complementary to a three-dimensional structure of a microorganism to be detected. The sensor detects a microorganism on the basis of a captured state of the microorganism onto the template. The polymer layer is formed by a manufacturing method including a polymerization step of polymerizing a monomer in the presence of a microorganism to be detected to form a polymer layer having the microorganism incorporated therein on the detection electrode, and a disruption step of bringing at least a part of the microorganism incorporated in the polymer layer into contact with a solution containing a lytic enzyme to disrupt the microorganism. According to a preferred embodiment of the sensor, the solution for use in the disruption step further contains a chelating agent.

A preferred embodiment of the sensor further includes a crystal oscillator which sets the detection electrode of the detection unit to be one electrode, and a captured state of the microorganism is detected by measuring a change in a mass of the polymer layer in accordance with a change in a resonance frequency of the crystal oscillator.

In the sensor described above, the monomer is preferably selected from the group consisting of pyrrole, aniline, thiophene, and derivatives of those. More preferably, the monomer is constituted of pyrrole or its derivative.

In the sensor described above, a surface of the detection electrode on which the polymer is formed is perferably a roughened surface.

In the sensor described above, the microorganism described above is preferably a microorganism in a state of having an excessive negative electric charge on its entirety or on its surface.

Moreover, the present invention is a method for manufacturing a sensor for detecting a microorganism, which is provided with a detection unit equipped with a detection electrode and a polymer layer, the polymer layer being arranged on the detection electrode and being provided with a template having a three-dimensional structure complementary to a three-dimensional structure of a microorganism, and the method includes a polymerization step of polymerizing a monomer in the presence of a microorganism to be detected to form the polymer layer having the microorganism incorporated therein on the detection electrode and a disruption step of bringing at least a part of the microorganism incorporated in the polymer layer into contact with a solution containing a lytic enzyme to disrupt the microorganism.

Moreover, the present invention is a polymer layer provided with a template having a three-dimensional structure complementary to a three-dimensional structure of a microorganism, and the polymer layer is manufactured by the manufacturing method including a polymerization step of polymerizing a monomer in the presence of the microorganism to form a polymer layer and a disruption step of bringing the microorganism incorporated in the polymer layer into contact with a solution containing a lytic enzyme to disrupt the microorganism.

### ADVANTAGEOUS EFFECTS OF INVENTION

According to the sensor of the present invention, a microorganism can be detected in a prompt and simple manner with a high sensitivity. Moreover, according to the method for manufacturing a sensor of the present invention, a sensor can be provided which can detect a microorganism in a prompt and simple manner with a high sensitivity.

### BRIEF DESCRIPTION OF DRAWINGS

Fig. 1 schematically represents preferable steps of manufacturing a polymer layer of a sensor according to the present invention, and (a) represents a cross-sectional view before a polymerization step, and (b) represents a cross-sectional view after the polymerization step, and (c) represents a cross-sectional view after a disruption step.
Fig. 2 is a schematic view representing a condition in which a target microorganism is captured onto a template in the sensor of the present invention, and (a) represents the case having a target microorganism, and (b) represents the case not having a target microorganism.
Fig. 3 is a schematic view representing a schematic configuration of a QCM sensor according to the present invention.
Fig. 4 is an electron micrograph representing Pseudomonas aeruginosa.
Fig. 5 is an electron micrograph representing a surface of a polypyrrole layer after the polymerization step in Example 1.
Fig. 6 is an electron micrograph representing a surface of a polypyrrole layer after being cleansed with sterile water after the disruption step of Example 1.
Fig. 7 is an electron micrograph representing a surface of a polypyrrole layer after being cleansed with sterile water after the disruption step of Example 2.
Fig. 8 is a graph representing a change in a resonance frequency of a crystal oscillator at the time of detection of a microorganism with use of the sensor of Example 1.
Fig. 9 is a graph representing a change in a resonance frequency of a crystal oscillator at the time of detection of a microorganism with use of the sensor of Example 2.

### DESCRIPTION OF EMBODIMENTS

A sensor of the present invention is provided with a detection unit equipped with a detection electrode and a polymer layer, wherein the polymer layer is arranged on the detection electrode and is provided with a template having a three-dimensional structure complementary to a three-dimensional structure of a microorganism, and the detects a microorganism on the basis of a captured state of the microorganism onto the template.

The polymer of the sensor of the present invention is formed by a manufacturing method including a polymerization step of polymerizing a monomer in the presence of a microorganism to be detected (hereinafter, also referred to as "target microorganism") to form the polymer layer having the microorganism incorporated therein on the detection electrode, and a disruption step of bringing at least a part of the microorganism incorporated in the polymer layer into contact with a solution containing a lytic enzyme to disrupt the microorganism.

Hereinafter, a preferred embodiment of the present invention will be described with reference to the drawings.

### [Producing Polymer Layer in Sensor]

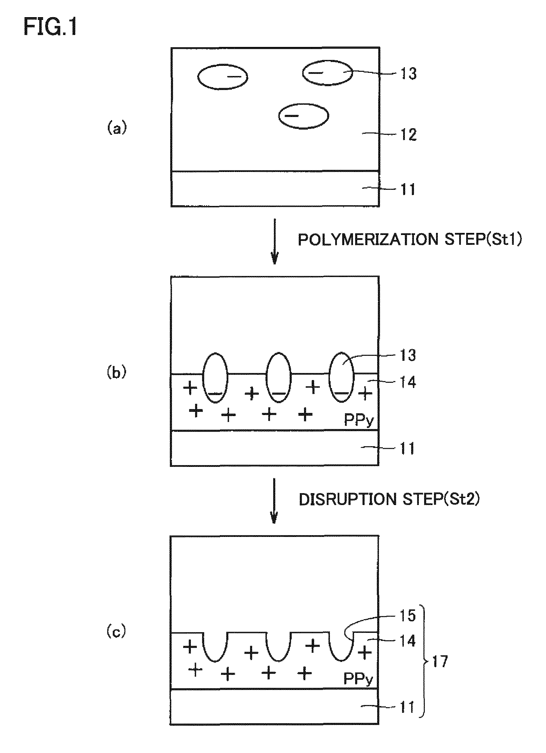

Fig. 1 is a cross-sectional view schematically representing preferable steps of manufacturing a polymer layer of a sensor according to the present invention. Fig. 1 represents an embodiment in which pyrrole is used as a monomer. Firstly, as shown in Fig. 1(a), a solution 12 containing microorganisms 13 and pyrrole under the environment in contact with a detection electrode 11. A concentration of the monomer constituting the polymer layer included in solution 12 can be 1 mM to 100M, and a concentration of microorganisms 13 can be 1 to 1x10¹⁰ cfu/mL.

In the polymerization step (St1), electrolysis is performed with detection electrode 11 as an anode and a counter electrode (not illustrated) as a cathode, and an oxidative polymerization reaction of pyrrole causes a polymer layer 14 constituted of polypyrrole ("PPy" in Fig. 1(b) is an abbreviation of polypyrrole) to be formed on detection electrode 11. Microorganisms 13 are incorporated into formed polymer layer 14. The pyrrole itself has a positive electric charge to discharge electrons to detection electrode 11 in the polymerization step. It is considered that microorganisms 13 in the state of having excessive negative electric charge on its entirety or on its surface are incorporated in polymer layer 14 to compensate for the positive electric charge.

Next, in the disruption step (St2), as shown in Fig. 1(c), the disruption step of disrupting microorganisms 13 incorporated in polymer layer 14 is performed. The disruption step can be performed by bringing microorganisms 13 into contact with a solution containing a lytic enzyme such as lysozyme. By the disruption step, microorganisms 13 are disrupted, and microorganisms 13 are ejected from polymer layer 14. Preferably, the solution used in the disruption step further contains a chelating agent. The chelating agent used in the disruption step may be EDTA (ethylenediaminetetraacetic acid), EGTA, NT A, DTPA, HEDTA, or the like. Moreover, the lytic enzyme used in the disruption step may be lysozyme, N-acetylmuramidase, Achromobacter endopeptidase, or the like.

By using the solution containing lytic enzyme, microorganisms 13 incorporated in polymer layer 14 can be disrupted, and disrupted microorganism 13 are ejected from polymer layer 14 to form a template 15. It is considered that the chelating agent contained in the solution facilitates the manifestation of bacteriolysis by lytic enzyme. Thus, containing the chelating agent can cause a degree of disruption of microorganisms 13 to be greater, so that it is understood that ejection of microorganisms from polymer layer 14 is facilitated with the disruption. By using the lytic enzyme and chelating agent, the efficiency of the disruption step can be readily improved, so that, as will be described later, the detection sensitivity of the sensor can be improved as compared to the case without the chelating agent. Preferably, the concentration of lytic enzyme in the solution used for the disruption step is 1 to 1000 mg/mL. When the chelating agent is added, it is preferable that the concentration of the chelating agent in the solution is 10 to 1000 µg/mL. In the bacteriolysis step, it is preferable that the time of bringing the solution containing the lytic enzyme into contact with microorganisms 13 incorporated in polymer layer 14 is twelve to forty-eight hours. It should be noted that, in the disruption step, the treatment of bringing the solution into contact with the microorganisms may be combined with a heating treatment, an ultrasonic treatment, and the like.

The region where microorganisms 13 were present in polymer layer 14 becomes template 15 having a three-dimensional structure complementary to the three-dimensional structure of microorganism 13. A detection unit 17 of the sensor according to the present invention is configured by a layered body constituted of polymer layer 14 provided with template 15 formed in such a manner and detection electrode 11. The thickness of polymer layer 14 in detection unit 17 can be, for example, 0.1 to 10 µm.

Microorganism 13 to be detected is not limited as long as it is a microorganism in the state of having an excessive negative electric charge on its entirety or on its surface, and various examples may be provided, such as Escherichia of colibacillus, Pseudomonas such as Pseudomonas aeruginosa, Acinetobacter such as Acinetobacter calocoaceticus, bacteria of Serratia, Klebsiella, Enterobacter, Citrobacter, Burkholderia, Sphingomonadase, Chromobacterium, Salmonella, Vibrio, Legionella, Campylobacter, Yersinia, Proteus, Neisseria, Staphylococcus, Streptococcus, Enterococcus, Clostridium, Corynebacterium, Listeria, Bacillus, Mycobacterium, Chlamydia, Rickettsia, Haemophilus, and the like. Moreover, various examples of virus may be provided such as hepatitis A virus, adenovirus, rotavirus, and norovirus, and an example of fungus may be provided such as Candida, and an example of protozoan may be provided such as Cryptosporidium. An electric charge on an entirety or on a surface of a microorganism is changed in accordance with a water quality of solution 12 such as pH. For example, various functional groups such as a carboxyl group, an amino group, a phosphate group, and the like are provided on a surface of a microorganism, and a surface including these functional groups is negatively charged when pH is high. Therefore, when forming a template or performing a measurement, solution 12 may be set to alkaline to obtain the state of having an excessive negative electric charge.

In Fig. 1, the case of using pyrrole as a monomer and forming a polypyrrole layer as a polymer layer is described. However, the monomer as a raw material of the polymer layer is not limited to pyrrole. Other material such as aniline, thiophene, and derivatives of those may be provided as examples.

The material of detection electrode 11 is not particularly limited, and various examples may be provided such as a gold electrode, a multilayer electrode of gold and chromium, a multilayer electrode of gold and titanium, a silver electrode, a multilayer electrode of silver and chromium, a multilayer electrode of silver and titanium, a lead electrode, a platinum electrode, a carbon electrode, and the like. It is preferable that a surface-roughening treatment is applied onto a surface of detection electrode 11 on which polymer layer 14 is formed. Forming a roughened surface on the surface of detection electrode 11 on which polymer layer 14 is formed, the effect of improving an adhesion with polymer layer 14 and expanding a surface area of the electrode can be obtained. For example, when a gold electrode is used as detection electrode 11, the surface-roughening step of applying a plasma-etching with respect to a gold electrode surface and fixing gold nanoparticles to apply a surface-roughening treatment can be performed. A surface roughness of detection electrode surface 11 can be, for example, a center line average roughness of 0.4 to 50 µm.

### [Capturing Target Microorganism onto Template]

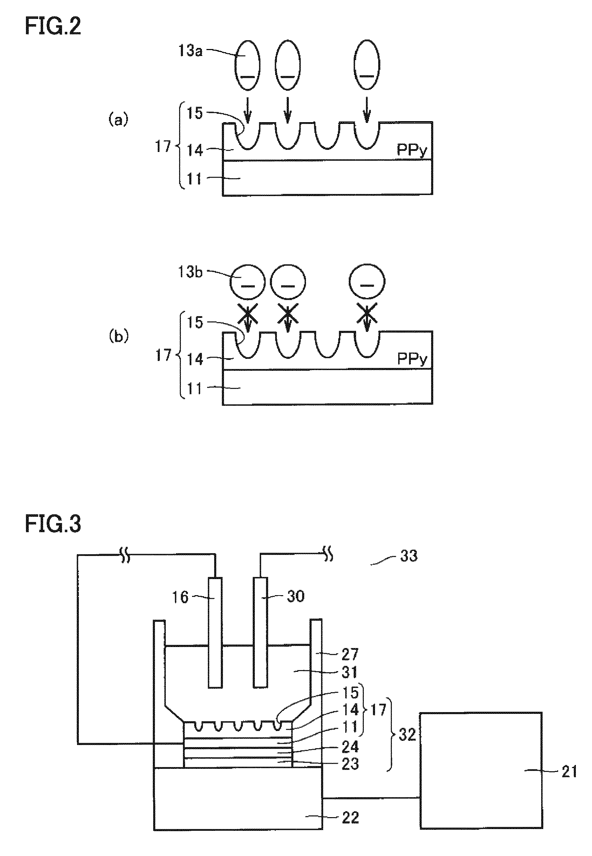

Fig. 2 is a schematic view representing a condition in which target microorganisms are captured onto the template. Fig. 2(a) represents the case where microorganisms 13a in a sample solution are target microorganisms, and Fig. 2(b) represents the case where microorganisms 13b in a sample solution are not target microorganisms. As shown in Figs. 2(a) and 2(b), firstly, a sample solution is prepared under the environment of bringing into contact with detection unit 17 constituted of polymer layer 14 and detection electrode 11. When negatively charged microorganisms move in the direction of detection unit 17 by electrostatic interaction with a positively charged PPy film or the like, microorganisms 13a having a three-dimensional structure complementary to a three-dimensional structure of template 15 are captured in template 15 (Fig. 2(a)), but microorganisms 13b not complementary to template 15 are not captured in template 15 (Fig. 2(b)). It should be noted that the movement of the microorganisms may be an active movement of the microorganism, or may be a movement by means of electropherogram, dielectrophoresis, stream, or simply precipitated or dispersed. Moreover, even in the case where suspended matters, such as mud and iron rust, other than microorganisms are included in water, since these matters have a three-dimensional shape and an electric charge state different from those of template 15 and not complementary, they are not captured. Therefore, a target microorganism can be distinguished from other suspended matters.

### [Detection of Target Microorganism]

When microorganisms 13a are captured into template 15, a layered body constituted of polymer layer 14 and detection electrode 11 goes under, for example, a mass change, a conduction characteristic change, an electric capacity change, a light reflectance change, a temperature change, and the like. In the sensor of the present invention, such changes are detected to detect a captured state of microorganisms into template 15. Then, the target microorganisms can be detected on the basis of the captured state. With such a detection, detection of a target microorganism in a prompt manner with a high sensitivity can be achieved. As a specific example of a method for detecting a mass change, a detection method of detecting a change in a resonance frequency of a crystal oscillator is included. Hereinafter, a crystal oscillator microbalance (QCM) sensor as a preferable example of the sensor of the present invention will be described.

### (QCM Sensor)

Fig. 3 is a schematic diagram representing a schematic configuration of the QCM sensor. QCM sensor 33 includes a cell 27 for retaining a solution, a crystal oscillator 32 arranged on a bottom portion of cell 27, an oscillating circuit 22, and a controller 21 having a frequency counter. Crystal oscillator 32 includes detection unit 17 produced by the method shown in Fig. 1, a crystal piece 24, and a counter electrode (second counter electrode) 23 layered in this order. QCM sensor 33 further includes a counter electrode (first counter electrode) 16 and a reference electrode 30 immersed in a sample solution 31, and a direct-current power supply can be connected between detection electrode 11 of detection unit 17 and counter electrode 16.

Firstly, sample solution 31 is added to cell 27. Then, oscillating circuit 22 applies an alternating-current voltage between detection electrode 11 and counter electrode 23 to oscillate crystal piece 24. When microorganisms are captured in template 15 of polymer layer 14, a change occurs in a mass of detection unit 17, and a resonance frequency of crystal piece 24 is changed. A frequency counter in controller 21 receives a signal from oscillating circuit 22 and measures a resonance frequency value. A captured state of the microorganisms is detected in accordance with a change in the resonance frequency value.

With use of QCM sensor 33 shown in Fig. 3, a polymer layer can be formed on detection electrode 11 in accordance with a surface-roughening treatment with respect to a surface of detection electrode 11 and the steps shown in Fig. 1. In these cases, a crystal oscillator having detection electrode 11, crystal piece 24, and counter electrode 23 layered in this order is arranged on a bottom portion of cell 27, and an alternating-current power supply is connected between detection electrode 11 and counter electrode 16. When forming the polymer layer with use of QCM sensor 33, a change in a resonance frequency of the crystal oscillator is monitored together at the time of forming a polymer layer, so that a progress of formation of the polymer layer can be confirmed. When plural kinds of microorganisms to be detected are present, a template of the present invention can be formed individually for each of those, or templates corresponding to a plurality of microorganisms can be concurrently formed in a single template to detect plural kinds of microorganisms at the same time.

According to the sensor of the present invention, bacteria can be detected in, for example, several minutes to several tens of minutes, and the detection can be performed far promptly as compared to the cultivation. Moreover, since detection can be performed without using a dyeing reagent required for a fluorescence cytology or an ATP extracting reagent required for measuring the number of bacteria with ATP, incorporation into equipment such as a water purifier, a water server, or a an automatic ice making device, or automation can be readily made. Moreover, as a bacteriological examination tool for a water quality examination and a food evaluation, it can be used in a water purifying plant or a drink/food factory. More specifically, measures can be taken such as automatically detecting bacteria in devices of a water storage tank or a piping route, notifying to a user, and automatically disinfecting and cleansing. Moreover, it can be incorporated as a device in a upper and lower piping lines in a water purifying plant to detect bacteria in water to be distributed.

The polymer layer in the sensor described above can be used not only as a constituting element of the sensor but also for a microorganism capturing device, a microorganism shape identifying device, or a microorganism tracking device utilizing a characteristic of having a template with a three-dimensional structure complementary to a three-dimensional shape of a microorganism, or for a catalyst carrier utilizing its characteristic as a porous body.

### Examples

Hereinafter, examples of the present invention will be described. The following examples illustrate the present invention, and do not limit the present invention.

In the following Examples 1 and 2, a polymer layer was produced with use of an electrochemical measuring system (Model 842B, manufactured by ALS Technology Co., Ltd), and Ag/AgCl (saturated KCl) was used for a reference electrode, and a Pt bar (a diameter of 1 mm, a length of 4 cm, manufactured by The Nilaco Corporation) was used as a counter electrode (first counter electrode). In the following description, an electric potential is provided which is a value with respect to an electric potential of this reference electrode. Moreover, a crystal oscillator (an electrode area of 0.196 cm², a basic oscillation frequency of 9 MHz, AT cut, a square shape, manufactured by Seiko EG&G Co., Ltd.) provided with gold electrodes (detection electrode and second counter electrode) on its both sides was used.

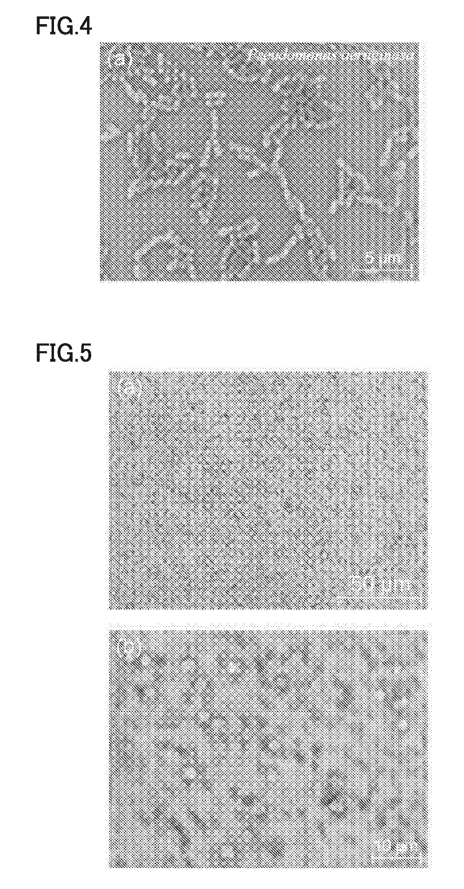

In Examples 1 and 2, Pseudomonas aeruginosa (zeta potential: -33.87mV) was used as a microorganism to be detected. Fig. 4 represents an electron micrograph of Pseudomonas aeruginosa.

### <Producing Sensor>

### [Example 1]

### (Surface-Roughening Step of Gold Electrode)

A surface-roughening treatment for a gold electrode surface of a crystal oscillator layered by was performed with respect to a surface of the gold electrode layer in accordance with the following procedures to improve an adhesion with a polypryrrole layer.
1. Etching was performed for 30 seconds with respect to a gold electrode (Product Name: QA-A9M-AU, manufactured by Seiko EG&G Co., Ltd.) by means of a a plasma etching device (SEDE/meiwa fosis).
2. A crystal oscillator was installed on a bottom portion of a cell (well-type cell, product name: QA-CL4, manufactured by Seiko EG&G Co., Ltds.) of QCM sensor 33 as shown in Fig. 3. After that, a solution of 500 µL containing citric acid protected gold nanoparticles (0.0574 wt%) of 30 nm was added to cell 27, and left for twenty-four hours at a room temperature.
3. After cleansing a gold electrode with purified water, a solution (Au nano particle growth solution) prepared by mixing a brominated hexadecyl trimethyl ammonium solution (0.1M) of 9 mL, gold chloride (III) acid tetrachloride (0.01M) of 250 µL, NaOH (0.1M) of 50 µL, and an ascorbic acid (0.1M) of 50 µL was added to cell 27, and left for twenty-four hours at a room temperature.
4. The solution in cell 27 was removed, and the gold electrode was cleansed with ultrapure water.

### (Producing Polypyrrole Layer Including Template of Microorganism)

A polypyrrole layer was produced on the gold electrode in accordance with the following procedures.
5. A pyrrole water solution of 0.1 M containing Pseudomonas aeruginosa of 1x10⁹ cfu/mL was produced to have a modification solution.
6. In cell 27 of QCM sensor 33 where the gold electrode is arranged to which the surface-roughening treatment was applied as described above, the modification solution was added, and the first counter electrode and the reference electrode were inserted into the modification solution.
7. A constant electric potential electrolysis (+0.975V, ninety seconds) was performed in the modification solution to precipitate Polypyrrole on the gold electrode, and produce a polypyrrole layer (polymerization step), and thereafter the polypyrrole layer was cleansed with sterile water. In the polymerization step, monitoring for the resonance frequency of the crystal oscillator was also performed. As to the polypyrrole layer after the polymerization step, a surface observation was performed by means of a scanning microscope (SEM). The thickness of the polypyrrole layer was 0.6 µm.
8. An EDTA solution (400 µg/mL, pH: 8.07, Tris buffer) was produced, and lysozyme was added to this EDTA solution and dissolved therein to prepare a lysozyme solution (20 mg/mL). Moreover, a Triton solution (20 wt%, pH 8.03, Tris buffer) containing a nonionic surfactant (product name: Triton) was also prepared together.
9. The produced lysozyme solution of 250 µL was added to cell 27, and left for one day at a room temperature. Further, the triton solution of 250 µL was dripped and left for one day at a room temperature to perform a bacteriolysis treatment of the microorganism incorporated in the polypyrrole layer (disruption step).
10. The solution in cell 27 was removed, and the polypyrrole layer was cleansed with sterile water. After that, the surface observation was performed with use of a scanning election microscope (SEM).

### [Example 2]

In the item "8." in Example 1 described above, lysozyme solution (20 mg/mL) not containing EDTA was prepared. In the item "9.", this lysozyme solution was used. Other than those, a polypyrrole layer was produced in a manner similar to that of Example 1.

### <Surface Observation for Polypyrrole Layer with Use of SEM>

Fig. 5 is an electron micrograph representing a surface of a polypyrrole layer after the polymerization step in Example 1. Fig. 5(b) is an electron micrograph representing an enlargement of a part of Fig. 5(a). In Fig. 5, a condition in which Pseudomonas aeruginosa was incorporated into a surface of the polypyrrole layer was observed.

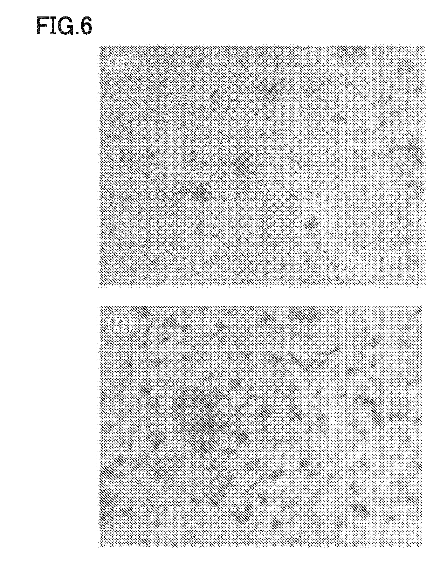

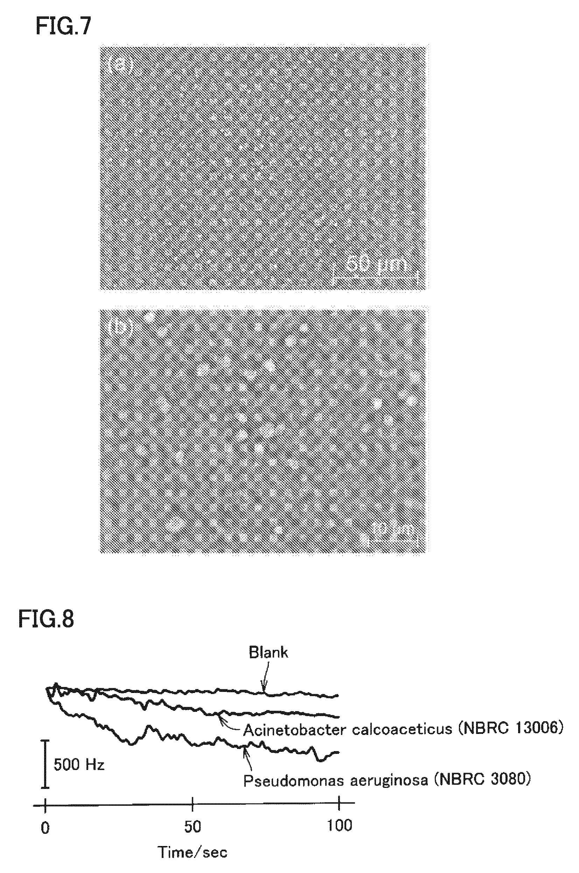

Figs. 6(a) and 6(b) are electron micrographs representing the surface of the polypyrrole layer cleansed with sterile water after the disruption step of Example 1, and Fig. 6(b) is an electron micrograph representing an enlargement of a part of Fig. 6(a). Figs. 7(a) and 7(b) are electron micrographs representing the surface of the polypyrrole layer cleansed with sterile water after the disruption step of Example 2, and Fig. 7(b) is an electron micrograph representing an enlargement of a part of Fig. 7(a). In Fig. 6, it can be found that, as compared with Fig. 7, almost no incorporated Pseudomonas aeruginosa is present, and Pseudomonas aeruginosa template is formed in the polypyrrole layer. It should be noted that it can be found also in Figs. 7(a) and 7(b) that, although Pseudomonas aeruginosa is present at a part of the surface of the polypyrrole layer, the template is formed at the same time.

### <Detection of Microorganism>

### (Detection Experiment)

Detection of microorganism was performed with use of the QCM sensor provided, at its bottom portion, with a crystal oscillator having a polypyrrole layer formed on the surface produced in the manner described above. A sample solution including microorganisms in the cell was added. Then, the resonance frequency of the crystal oscillator was monitored.

### (Result)

Fig. 8 is a graph representing a change in a resonance frequency of a crystal oscillator in the sensor of Example 1. From the result shown in Fig. 8, it could be found that in the sensor of Example 1, when a sample solution including Pseudomonas aeruginosa was added, as compared with a sample including Acinetobacter (Acinetobacter calcoaceticus) or a blank, the resonance frequency was reduced significantly. The reduction in the resonance frequency means an increase in the mass of the crystal oscillator surface, and it can be considered that the mass of the crystal oscillator surface increased by incorporation of Pseudomonas aeruginosa into the template of the polypyrrole layer. Thus, it can be found that Pseudomonas aeruginosa can be detected with the sensor of Example 1.

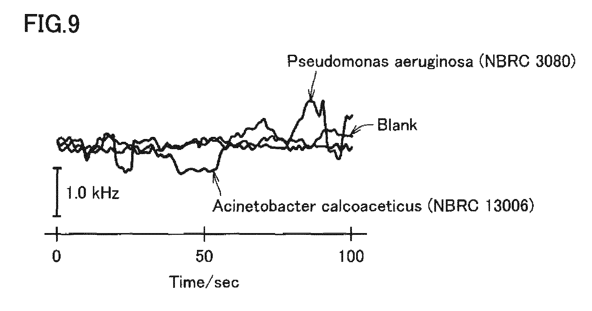

Fig. 9 is a graph representing a change in a resonance frequency of the crystal oscillator in the sensor of Example 2. From the result shown in Fig. 9, in the sensor of Example 2, no reduction in the resonance frequency could be found even when the sample solution including Pseudomonas aeruginosa was added. However, since it can be found from Figs. 7(a) and 7(b) that the template is formed in the surface of the polypyrrole layer, it can be understood that, when a detection procedure having a higher sensitivity than the detection procedures used herein is employed, the sensor of Example 2 can also detect Pseudomonas aeruginosa.

With use of the sensor according to the present invention, microorganisms can be detected in a prompt and easy manner, for example, in a food-processing factory, not only it contributes to the reduction of the defective goods rate and the reduction of the storage cost but also grasping of a contamination pathway of microorganisms and a proposal for a measure can be readily provided by forming a desired template of a microorganism and detecting the microorganism.

### REFERENCE SIGNS LIST

11 detection electrode; 12 solution; 13 microorganism; 14 polymer layer; 15 template; 16 counter electrode (first counter electrode); 17 detection unit; 21 controller; 22 oscillating circuit, 23 counter electrode (second counter electrode); 24 crystal piece; 27 cell; 30 reference electrode; 31 sample solution; 32 crystal oscillator; 33 QCM sensor.

## Claims

1. A sensor comprising:
a detection unit equipped with a detection electrode and a polymer layer, said polymer layer being arranged on said detection electrode and being provided with a template having a three-dimensional structure complementary to a three-dimensional structure of a microorganism to be detected,
said sensor being configured to detect said microorganism on the basis of a captured state of said microorganism onto said template,
said polymer layer being formed by a manufacturing method including a polymerization step of polymerizing a monomer in the presence of a microorganism to be detected to form said polymer layer having said microorganism incorporated therein on said detection electrode, and a disruption step of bringing at least a part of said microorganism incorporated in said polymer layer into contact with a solution containing a lytic enzyme to disrupt said microorganism.

2. The sensor according to claim 1, wherein said solution used in said disruption step further contains a chelating agent.

3. The sensor according to claim 1 or 2, further comprising:
a crystal oscillator which sets said detection electrode of said detection unit to be one electrode, wherein
a captured state of said microorganism is detected by measuring a change in a mass of said polymer layer in accordance with a change in a resonance frequency of said crystal oscillator.

4. The sensor according to any one of claims 1 to 3, wherein said monomer is selected from the group consisting of pyrrole, aniline, thiophene, and derivatives of those.

5. The sensor according to claim 4, wherein said monomer is constituted of pyrrole or its derivative.

6. The sensor according to any one of claims 1 to 5, wherein a surface of said detection electrode on which said polymer layer is formed is a roughened surface.

7. The sensor according to any one of claims 1 to 6, wherein said microorganism is in a state of having an excessive negative electric charge on its entirety or on its surface.

8. A method for manufacturing a sensor for detecting a microorganism, said sensor being provided with a detection unit equipped with a detection electrode and a polymer layer, said polymer layer being arranged on said detection electrode and being provided with a template having a three-dimensional structure complementary to a three-dimensional structure of a microorganism, said method comprising:
a polymerization step of polymerizing a monomer in the presence of a microorganism to be detected to form said polymer layer having said microorganism incorporated therein on said detection electrode; and
a disruption step of bringing at least a part of said microorganism incorporated in said polymer layer into contact with a solution containing a lytic enzyme to disrupt the microorganism.

9. A polymer layer provided with a template having a three-dimensional structure complementary to a three-dimensional structure of a microorganism, said polymer layer being manufactured by a manufacturing method including:
a polymerization step of polymerizing a monomer in the presence of said microorganism to form the polymer layer; and
a disruption step of bringing at least a part of the microorganism incorporated in said polymer layer into contact with a solution containing a lytic enzyme to disrupt the microorganism.
